# NEW EUROPEAN PATENT SPECIFICATION

(11) **EP 0 211 984 B2**
(45) Date of publication and mention of the opposition decision: **11.09.1996**
(45) Mention of the grant of the patent: 10.06.1992
(21) Application number: 85110377.0
(22) Date of filing: 19.08.1985
(51) Int. Cl.: G06K 11/18, G01D 5/26, A61B 5/02

(54) **Computer data entry and manipulation apparatus**
Apparat zur Eingabe und Behandlung von Computerdaten
Appareil pour l'introduction et la manipulation des données d'ordinateurs

(43) Date of publication of application: 04.03.1987
(73) Proprietor: VPL RESEARCH, INC., Foster City, CA 94404 (US)
(72) Inventor: VPL RESEARCH, INC., Foster City, CA 94404 (US)
(74) Representative: Chaverneff, Vladimir

(56) References cited:
- EP-A- 0 121 387
- EP-A- 0 123 043
- DE-A- 3 334 395
- US-A- 3 510 210
- US-A- 4 396 885
- US-A- 4 414 537
- US-A- 4 524 348
- US-A- 4 542 291
- The thesis by Paul Jerome Kilpatrick"The use of a kinesthetic supplement in an interactive graphics system", made publicly available by University Microfilm International on 15 February 1977, catalogued as UMI 7702061. Issue 37, Volume 8B.
- The paper "A three-dimensional computer graphics workstation" published by Pergamon Infotech Ltd. in "Computer-aided design and manufacture, State of the Art Report 13:8", in 1985.
- The edition of the book "Artificial Reality" by Myron W. Krüger, pages 105-108, and 147, published in USA in 1983 by Addison-Wesley Publishing Company Inc.

## Description

### BACKGROUND OF THE INVENTION

### Field of the Invention

This invention relates generally to the field of devices for data entry and manipulation in computers, and relates more particularly to an apparatus for entering data into a computer and manipulating virtual objects defined by the computer based on the gestures and positions of the hand of an operator.

### Description of the Prior Art

Input devices for computers include such devices as keyboards, digitizers, joysticks, mice, trackballs, and light pens. One function of these input devices is to position, in two dimensions, a cursor on the display screen of a computer. Once the cursor is positioned at a desired location, the computer is instructed to perform an operation. The processes of cursor positioning and operation selection are discrete operations, since separate motions are required to perform each operation. With a mouse, for example, cursor positioning is accomplished by moving the mouse along a surface, while operation selection is accomplished by pushing keys located either on the mouse or a separate keyboard. The mastering of the operation of such input devices is often difficult because the hand movements required to operate the devices do not correspond to the visual feedback presented by the display screen of the computer.

Glove input devices have been used to input data to computers. U.S. Patent No. 4,414,537, issued November 8, 1983 to G. Grimes and entitled "Digital Data Entry Glove Interface," describes one such glove input device. The Grimes patent discloses a glove with sensors for detecting the flexing of finger joints, sensors for detecting contact between various portions of the hand, and sensors for detecting the orientation of the hand. The Grimes device is adapted for the identification of static hand positions that represent the alphanumeric characters of the manual alphabet.

### SUMMARY OF THE INVENTION

The present invention which is defined by the claims provides an apparatus for entering data into a computer and operates in accordance with the following principles. The apparatus manipulats virtual objects defined by a computer according to the gestures and positions of the hand of an operator. Such manipulation includes positioning a cursor or other representation of the hand of the operator with respect to virtual objects defined by the computer according to certain position specifying movements of the operator's hand, and operating on those virtual objects according to certain gesture specifying movements of the operator's hand. Gesture sensing means are coupled to the hand for detecting the gesture specifying movements of the hand, hand position sensing means also coupled to the hand at least in part for detecting the position specifying movements of the hand, and signal processing means adapted to receive data from the gesture sensing means and the hand position sensing means and to instruct the computer to manipulate virtual objects according to the movements of the operator's hand.

The gesture sensing means includes a glove assembly with attached sensors that are responsive to the degree of flex of the fingers of the operator's hand. These flex sensors are mounted on a flexible printed circuit board and are sandwiched between an inner and an outer glove. A decoding circuit for addressing the sensors is also mounted on the flexible printed circuit board, and is electrically coupled to the sensors through the flexible printed circuit board and to the computer via a detachable cable. The hand position sensing means includes a mobile ultrasonic transmitter affixed to the glove assembly, a stationary receiver comprising three separate spaced-apart ultrasonic receiving units, and a control circuit that measures the time delay of pulsed ultrasonic signals from the transmitter to the three receivers to indicate the spatial position of the operator's hand. The signal processing means is embodied in software, and includes routines for positioning a hand-shaped cursor on the display screen of the computer according to the position of the operator's hand, for responding to the output signals of the flex sensors to identify gestures of the operator's hand, and for manipulating virtual objects defined by the computer according to commands represented by the gestures and movement of the operator's hand.

The present invention enables the static and dynamic gestures of an operator's hand and the spatial position of the hand to be determined. As an input device, the present invention is especially well adapted for use with a pictorial or symbolic programming language having a dynamic cursor which corresponds in shape to the shape of the glove and moves on the screen in response to movement of the glove in space. The present invention provides the basis for a symbolic programming language in which the physical gestures of the operator's hand are used to implement conceptually similar and easily recognizable functions or operations on virtual objects displayed on the display screen of the computer.

### IN THE DRAWINGS

Figure 1 is an overall perspective view of a computer incorporating a computer data entry and manipulation apparatus according to the present invention;
Figure 2 is a back view of one embodiment of an instrumented glove assembly according to the present invention;
Figure 3 is a side view of the instrumented glove assembly of Figure 2;
Figure 4 is a palm side view of the instrumented glove assembly of Figure 2;
Figure 5 is a sectional detail view of one finger of the instrumented glove assembly of Figure 2, with the finger having an extended orientation;
Figure 6 is a sectional detail view of one finger of the instrumented glove assembly of Figure 2, with the finger having a bent orientation;
Figure 7 is a back view of an alternative embodiment of an instrumented glove assembly according to the present invention;
Figure 8 is a side view of the instrumented glove assembly of Figure 7;
Figure 9 is a palm side view of the instrumented glove assembly of Figure 7;
Figure 10 is a longitudinal cross-section view of one embodiment of the flex sensor;
Figure 11 is a longitudinal cross-section view of another embodiment of the flex sensor;
Figure 12 is a cross-sectional view through the structure shown in Figure 11;
Figure 13 is an alternate cross-sectional view through the structure shown in Figure 11.

### DETAILED DESCRIPTION OF THE PREFERRED EMBODIMENTS

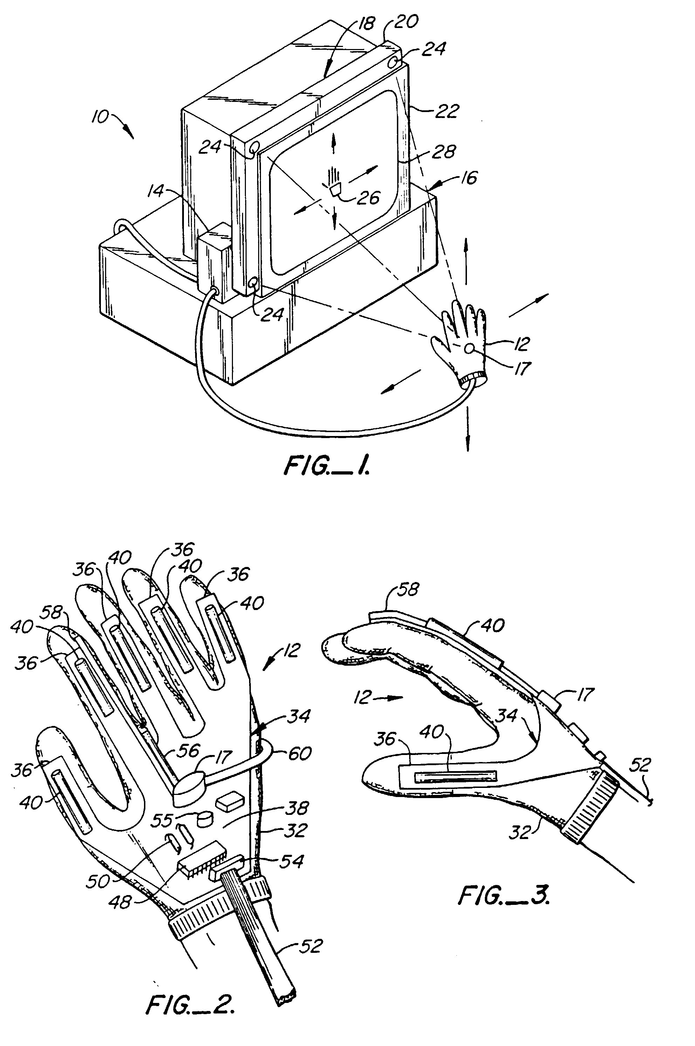

In Figure 1, the preferred embodiment of the present invention is illustrated in its intended mode of use, namely as a computer data entry and manipulation apparatus 10. The apparatus 10 includes a glove assembly 12 that is electrically coupled to an interface circuit 14 that is in turn electrically coupled to a computer 16, a position sensing receiver assembly 18 that is also electrically coupled to the interface circuit 14, and software that is executed by the computer 16. The glove assembly 12 contains sensors that detect the flexing of the fingers and other gestures of the hand of an operator, and also contains an ultrasonic transducer 17 used in detecting the spatial position of the glove assembly 12. The position sensing receiver assembly 18 includes an L-shaped frame 20 that preferably extends across the top and down one side of a monitor 22 of the computer 16. Three ultrasonic transducers 24 are located at the corner and ends of the frame 20 and face toward the operator.

In operation, the glove assembly 12 is worn on the hand of an operator, and is used to position a cursor 26 or other representation of the glove assembly on the display screen 28 of the computer 16. The spatial position of the glove assembly 12 is determined by transmitting an ultrasonic signal through the transducer 17 in the glove assembly, receiving that signal by the transducers 24 of the position sensing receiver assembly 18, and measuring the time delay of the signal from the transmitting transducer 17 to each of the receiving transducers 24. The software portion of the apparatus 10 converts the time delay data into orthogonal coordinates, and directs the computer 16 to display the cursor 26 on the display screen 28 accordingly. Thus, movement by the glove assembly 12 in a plane parallel to that of the display screen 28 results in corresponding movement by the cursor 26. Movement by the glove assembly 12 toward and away from the display screen 28 can be represented by varying the size of the cursor 26.

The glove assembly 12 is also used to enter commands into the computer 16. As described in detail below, the glove assembly 12 contains several sensors that respond to the gestures of the operator's hand. The software portion of the apparatus 10 receives and interprets gesture indicating data from the sensors of the glove assembly 12 and enters commands into the computer 16 according to the gestures recognized. These commands relate to the manipulation of virtual objects created by the computer 16 and displayed on the display screen 28.

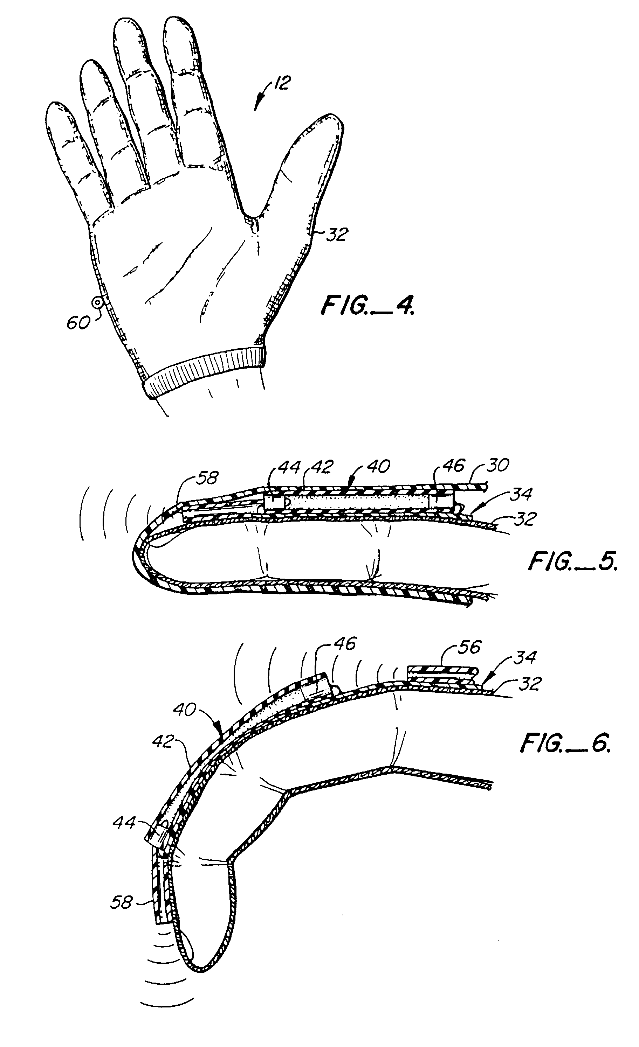

In Figures 2, 3, 4, 5, and 6, the preferred embodiment of the glove assembly 12 is illustrated. An outer glove 30, shown in Figure 5, which protects the circuitry attached to the inner glove is not shown in Figures 2, 3, 4, and 6 to allow the apparatus to be more clearly depicted. The component parts of the glove assembly 12 are bonded or otherwise secured to an inner glove 32, which is worn on the hand of the operator during operation. In the illustrated embodiment, the sensors and electrical components of the glove assembly are soldered to and electrically interconnected by a flexible printed circuit board 34, which is itself bonded or otherwise secured to the inner glove 32. The flexible printed circuit board 34 includes five elongated portions 36 positioned along the back side of the fingers and thumb and extending from a central portion 38 positioned along the back of the hand. Preferably, the inner glove 32 is composed of a material such as stretch knitted nylon which accommodates various sizes of hands and maintains a good fit during use. The outer glove 30 covers and protects the components of the glove assembly 12, and improves the aesthetics of the glove assembly. Preferably, the outer glove 30 is composed of a light weight and durable material such as cotton.

The glove assembly 12 includes five flex sensors 40, each positioned on the back side of the inner glove 32 opposite a middle knuckle of one finger or thumb. The flex sensors 40 are preferably of the type that will provide a signal that is an analog representation of the degree of bend of each of the fingers and thumb. As shown in Figures 5 and 6, the flex sensor 40 comprises a flexible tube 42 having interior reflective walls with a light source 44 at one end and a photosensitive detector 46 at the other end. The light source 44 is preferably an infrared light emitting diode, and the photosensitive detector 46 is preferably a phototransistor. The flexible tube 42 is bonded or otherwise secured to the flexible printed circuit board 34, with the electrical leads of the light source 44 and the photosensitive detector 46 soldered to appropriate conductive traces of the flexible printed circuit board. The amount of light that impinges on the photosensitive detector 46, and the corresponding amount of current flowing through the photosensitive detector, is dependent upon the amount of bend of the flexible tube 42. When the finger is extended, as shown in Figure 5, the flexible tube 42 is generally straight and a maximum amount of light from the light source 44 impinges on the photosensitive detector 46. When the finger is flexed to a bent position, as shown in Figure 6, the center of the flexible tube 42 is almost pinched off, which restricts the amount of light transmitted to the photosensitive detector 46. Thus, the flex sensors 40 provide an analog signal that indicates the flexing of the operator's fingers and thumb.

The glove assembly 12 is electrically connected to the interface circuit 14 via a cable 52, which is preferably a flat ribbon cable that is releasably attached to the flexible printed circuit board 34 by a connector 54. The cable 52 also supplies power and ground signals to the components of the glove assembly 12.

As mentioned above, the glove assembly 12 includes an ultrasonic transducer 17 that transmits ultrasonic signals for use in determining the spatial position of the glove assembly. The ultrasonic transducer 17 is soldered to appropriate conductive traces at the central portion area 38 of the flexible printed circuit board 34, and is electrically connected to the interface electronics 14 via the cable 52. Preferably, components 55 including a transformer and transistor for driving the ultrasonic transducer 17 are also contained in the glove assembly 12 and mounted to the flexible printed circuit board 34. In order to counteract possible blocking of the ultrasonic signals by the operator's hand, three flexible transmission tubes 56, 58, and 60 are utilized to conduct the ultrasonic signals to different parts of the glove assembly. Transmission tubes 56 and 58 extend from the ultrasonic transducer 17 toward the index finger of the glove assembly, with transmission tube 56 ending at the base of the finger and transmission tube 58 extending nearly to the tip of the finger. Transmission tube 60 extends from the ultrasonic transducer 17 around the side of the glove assembly, as shown in Figure 4. Each of the transmission tubes carries the ultrasonic signals transmitted by the ultrasonic transducer 17 and radiates those signals out an open end. The transmission tubes ensure that hand gestures do not block the transmission of the ultrasonic signals to the position sensing receiver assembly 18. When, for example, the operator's index finger is pointing toward the display screen 28, as shown in Figure 5, the ultrasonic signals radiating from the end of the transmission tube 58 will reach the receiving transducers 24. When the operator's index finger is bent downward, as shown in Figure 6, the ultrasonic signals radiating from the end of the transmision tube 56 will reach the receiving transducers 24. When the operator's hand is positioned with the palm toward the display screen 28, the ultrasonic signals radiating from the end of the transmission tube 60 will reach the receiving transducers 24.

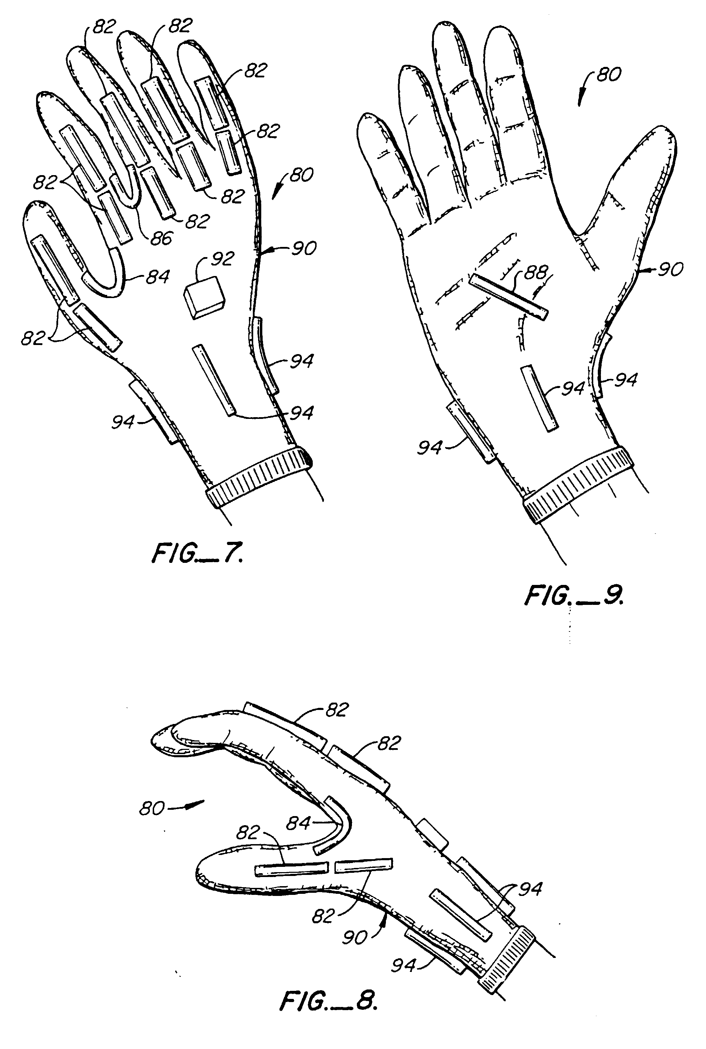

A more extensively instrumented alternative glove assembly 80, illustrated in Figures 7, 8, and 9, provides additional data for gesture identification. A flexible printed circuit board and the various electronic components of the glove assembly 80 are not shown for clarity, but may, of course, be included. The glove assembly 80 includes two flex sensors 82 for each finger and thumb, a flex sensor 84 between the thumb and index finger, a flex sensor 86 between the index finger and the middle finger, and a flex sensor 88 across the palm, all secured to an inner glove 90. The flex sensors 82 are located opposite the middle knuckle and the base knuckle of each finger and thumb. Flex sensor 84 is responsive to lateral movement of the thumb relative to the index finger, while flex sensor 86 is responsive to lateral movement of the index finger relative to the middle finger. The flex sensor 88 located across the palm is useful in detecting gestures such as touching the thumb to the little finger.

In addition to the flex sensors, the glove assembly 80 may also include a hand orientation sensor 92 which provides data indicative of the orientation of the glove assembly relative to the three rotational axis of roll, pitch, and yaw. The orientation sensor 92 can be implemented in various ways, such as a three-axis accelerometer, an array of mercury potentiometers, or a bubble gauge.

As an alternative to the use of spatial positioning of the glove assembly 12 for directing the two-dimensional positioning of the screen cursor 26, wrist motions may be used. For example, the forward and back flexing of the wrist can indicate vertical positioning of the screen cursor 26, while left and right flexing of the wrist can indicate horizontal positioning of the screen cursor. To this end, four additional flex sensors 94 are secured to the inner glove of the glove assembly 80 at locations surrounding the wrist joint.

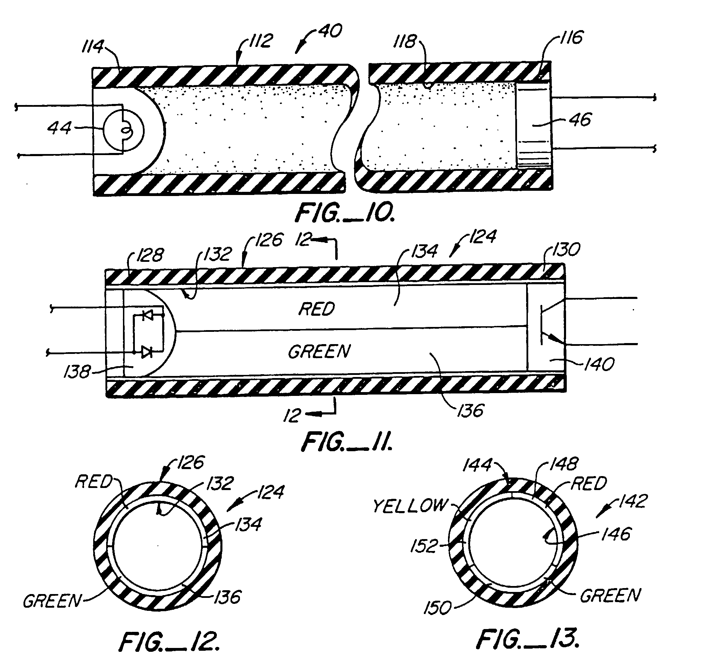

Figure 10 illustrates in detail one implementation of the optical flex sensor employed in the glove depicted in other figures. The sensor 40 consists of a flexible tube 112 that has open ends 114 and 116, a reflective interior wall 118 within the flexible tube 112, and a light source 44 placed at end 114. A photosensitive detector 46, or light transmitting device such as an optical fiber, is placed at end 116 of tube 112 so that the intensity of a combination of direct light rays and reflected rays may be detected when the flexible tube 112 is bent.

The flexible tube 40 may be fabricated from rubber, plastic, woven material, or other suitable flexible material, while the interior wall 118 may be coated with aluminum paint or other suitable reflective material, or in some instances left untreated.

In its unflexed position, the tube 112 is generally straight. In this position the light emitted from light source 44 strikes the photosensitive detector 46. As tube 112 is bent, the light received is a combination of direct light rays and reflected rays. The amount of light reaching the photosensitive detector 46 will decrease until a position is reached in which all of the light is blocked off or reflected, depending upon the resiliency of tube 112.

In one embodiment photosensitive detector 46 changes its resistance with light intensity. The combined effect of the bent tube 112 on the light path and the photosensitivity of the detector 46 results in a device which changes its electrical resistance (or other property) when flexed. Detector 46 may be a phototransistor, photosilicon-controlled rectifier, a photo cell, or an optical fiber which carries the signal to another location. In general detector 46 may be any component which has an output parameter which changes in response to light intensity.

Figure 11 illustrates another embodiment of an optical flex sensor 124. Sensor 124 consists of a flexible tube 126 which has two ends 128 and 130, and a reflective interior wall 132. Interior wall 132 is constructed from two different longitudinal color areas--red 134 and green 136. A light source 138, for example, light-emitting diodes or infrared emitters, is positioned at one end of the tube and a photosensitive detector 140, for example, a phototransistor, is positioned at the other end 130.

Figure 12 is a cross-sectional view of the tube shown longitudinally in Figure 11.

Another embodiment of the optical flex sensor 142 is depicted in Figure 13. The sensor shown has within flexible tube 144 a reflective interior wall 146 made from three different longitudinal color areas--red 148, green 150, and yellow 152. The two different color areas in the sensor of Figure 12 and the three different color areas in the sensor of Figure 13 cause the intensity of the light which reaches the photosensitive detector at the opposite end of the tube to be modified according to whether a light source of similar color or different color is reflected from the surface.

In each of these embodiments, the light source may consist of the same multiple number of similar colored sources as there are colored wall areas on the wall of the tube. These multiple sources may be pulsed on and off at various time intervals, and the output parameter of the detector at the opposing end of the tube sampled during corresponding time intervals. In this manner the information present allows determination not only of the degree that the device is bent, but also the direction of bending. The accuracy obtainable with respect to the direction of bending of the device will generally increase with the larger number of multiple colored light sources and correspondingly colored walls. Although only the specific case of one, two and three colors are illustrated, any desired number may be chosen.

## Claims

1. An apparatus for entering data into a computer according to gestures of the hand of an operator, comprising gesture sensing means including sensors adapted to be fixed to the hand, for detecting gesture specifying flexing movements within the hand and signal processing means coupled to said gesture sensing means for generating control signals according to said gesture specifying flexing movements,
characterized by the fact that said sensors are fixed in a glove (12), said sensors being of the type that provides a signal that is an analog representation of their degree of bend, and said sensors comprising at least one flex sensor for each finger and thumb (36, 40, 82), a flex sensor between the thumb and index finger (84), a flex sensor between the index finger and the middle finger (86) and a flex sensor across the palm (88)
and characterized by the fact that it comprises a transducer (17) fixed on the glove and a position sensing receiver assembly (18) for sensing the spatial position of said glove, said signal processing means being also coupled to said position sensing means (17,24) to move a cursor (26) in accordance with said position specifying movements of the hand,
said signal processing means further controlling said cursor (26) so as to implement functions or operations on virtual objects in accordance with said gesture specifying flexing movements of the hand.

2. The apparatus of claim 1, characterized by the fact that it comprises a hand orientation sensor (92).

3. The apparatus of claim 1 or 2, characterized by the fact that it comprises additional flex sensors (94) secured at locations surrounding the wrist joint.

4. The apparatus of any one of claims 1 to 3 wherein said gesture sensing means (40) provide a signal that is an analog representation of the degree of bend of a finger.

5. The apparatus of any one of claims 1 to 4, including display means (22) connected to the computer (16) for displaying said virtual object and said cursor (26) on a screen (28), said signal processing means being adapted to move said cursor (26) on said screen (28) in accordance with said position sensing means (17, 24).

6. The apparatus of claim 5, including means for displaying the cursor (26) as a representation of a hand which corresponds to the position and gesture of the operator's hand.

7. The apparatus of claim 5 or 6, wherein said signal processing means includes means for varying the size of said cursor (26) in accordance with movement of the hand toward and away from said display means (22).

8. The apparatus of any one of claims 1 to 7, wherein said position sensing means includes ultrasonic receivers (24) disposed on said display means (22), and means (17, 56, 58, 60) for transmitting ultrasonic signals from different locations of the operator's hand.

## Patentansprüche

1. Vorrichtung zum Eingeben von Daten in einen Computer entsprechend den Gesten der Hand einer Bedienungsperson, mit einem Gesten-Fühlermittel mit an der Hand befestigbaren Sensoren zum Erfassen von Gesten, die Biegebewegungen innerhalb der Hand ausdrücken, und mit Signalverarbeitungsmitteln, die an die Gestenfühlermittel angeschlossen sind, um Steuersignale entsprechend den Biegebewegungen ausdrückenden Gesten zu erzeugen, gekennzeichnet durch die Tatsache, daß die Sensoren in einem Handschuh (12) befestigt sind, wobei die Sensoren Sensoren des Typs sind, der ein Signal erzeugt, das eine analoge Darstellung ihres Biegegrads ist, wobei die Sensoren wenigstens einen Biegesensor für jeden Finger und den Daumen (36, 40, 82), einen Biegesensor zwischen dem Daumen und dem Zeigefinger (84), einen Biegesensor zwischen dem Zeigefinger und dem Mittelfinger (86) und einen Biegesensor über dem Ballen (88) enthalten, und gekennzeichnet durch die Tatsache, daß sie einen an dem Handschuh befestigten Wandler (17) und eine Positionserfassungs-Empfängerandordnung (18) zum Erfassen der räumlichen Position des Handschuhs enthält, wobei die Signalverarbeitungsmittel auch mit den Positionserfassungsmitteln (17, 24) verbunden sind, um einen Cursor (26) entsprechend den Positionsangabebewegungen der Hand zu bewegen, wobei die Signalverarbeitungsmittel außerdem den Cursor (26) so steuern, daß Funktionen oder Operationen an virtuellen Objekten entsprechend den Gesten angebenden Biegebewegungen der Hand zur Ausführung bringen.

2. Vorrichtung nach Anspruch 1, gekennzeichnet durch die Tatsache, daß sie einen Handlagesensor (92) enthält.

3. Vorrichtung nach Anspruch 1 oder 2, gekennzeichnet durch die Tatsache, daß sie zusätzliche Biegesensoren (94) enthält, die an Stellen um das Handgelenk herum befestigt sind.

4. Vorrichtung nach einem der Ansprüche 1 bis 3, bei welchem das Gestenfühlermittel (40) ein Signal liefert, das eine analoge Darstellung des Grads der Biegung eines Fingers ist.

5. Vorrichtung nach einem der Ansprüche 1 bis 4 mit einem Anzeigemittel (22), das zur Anzeige des virtuellen Objekts und des Cursors (26) auf einem Bildschirm (28) mit dem Computer (16) verbunden ist, wobei die Signalverarbeitungsmittel den Cursor (26) auf dem Bildschirm (28) entsprechend den Positionsfühlermitteln (17, 24) bewegen können.

6. Vorrichtung nach Anspruch 5, mit Mitteln zum Wiedergeben des Cursors (26) als eine Darstellung einer Hand, die der Position und der Geste der Hand der Bedienungsperson entspricht.

7. Vorrichtung nach Anspruch 5 oder 6, bei welcher das Signalverarbeitungsmittel ein Mittel zum Ändern der Größe des Cursors (26) entsprechend der Bewegung der Hand zu dem Anzeigemittel (22) oder davon weg enthält.

8. Vorrichtung nach einem der Ansprüche 1 bis 7, bei welcher das Positionsfühlermittel Ultraschallempfänger (24) enthält, die an dem Anzeigemittel (22) angeordnet sind, sowie Mittel (17, 56, 58, 60) zum Übertragen von Ultraschallsignalen von verschiedenen Stellen der Hand der Bedienungsperson aus enthält.

## Revendications

1. Appareil d'introduction de données dans un ordinateur en fonction des gestes de la main d'un opérateur, comprenant des moyens de détection des gestes comportant des détecteurs conçus pour être fixés sur la main, pour détecter des mouvements de flexion caractéristiques des gestes de la main et des moyens de traitement de signaux couplés auxdits moyens de détection des gestes pour produire des signaux de commande en fonction desdits mouvements de flexion caractéristiques de gestes,
caractérisé par le fait que lesdits capteurs sont fixés dans un gant (12), lesdits capteurs étant du type qui produit un signal qui est une représentation analogique de leur degré de flexion, et lesdits capteurs comprenant au moins un capteur de flexion pour chaque doigt et pour le pouce (36, 40, 82), un capteur de flexion entre le pouce et l'index (84), un capteur de flexion entre l'index et le majeur (86) et un capteur de flexion en travers de la paume (88),
et caractérisé par le fait qu'il comprend un transducteur (17) fixé sur le gant et un ensemble de réception de détection de position (18) pour détecter la position dans l'espace dudit gant,
ledits moyens de traitement de signaux étant aussi couplés audit moyen de détection de position (17, 24) pour déplacer un curseur (26) en fonction desdits mouvements caractéristiques de la position de la main,
lesdits moyens de traitement de signaux contrôlant en plus ledit curseur (26) de façon à mettre en oeuvre des fonctions ou des opérations sur des objets virtuels en fonction desdits mouvements de flexion de la main caractéristiques de gestes.

2. Appareil selon la revendication 1, caractérisé par le fait qu'il comprend un capteur d'orientation de la main (92).

3. Appareil selon la revendication 1 ou 2, caractérisé par le fait qu'il comprend d'autres capteurs de flexion (94) fixés à des endroits entourant l'articulation du poignet.

4. Appareil selon l'une quelconque des revendications 1 à 3, dans lequel ledit moyen de détection des gestes (40) produit un signal qui est une représentation analogique du degré de courbure d'un doigt.

5. Appareil selon l'une quelconque des revendications 1 à 4, comprenant aussi un moyen de visualisation (22) relié à l'ordinateur (18) pour visualiser ledit objet virtuel et ledit curseur (26) sur un écran (28), lesdits moyens de traitement de signaux étant conçus pour déplacer ledit curseur (26) sur ledit écran (28) en fonction dudit moyen de détection de position (17, 24).

6. Appareil selon la revendication 5, comportant un moyen de visualisation du curseur (26) sous forme d'une représentation de la main qui correspond à la position et au geste de la main de l'opérateur.

7. Appareil selon la revendication 5 ou 6, dans lequel lesdits moyens de traitement de signaux comprennent un moyen pour modifier les dimensions dudit curseur (26) en fonction du mouvement de la main se rapprochant ou s'éloignant dudit moyen de visualisation (22).

8. Appareil selon l'une quelconque des revendications 1 à 7, dans lequel ledit moyen de détection de la position comporte des récepteurs ultrasonores (24) disposés sur ledit moyen de visualisation (22), et des moyens (17, 56, 58, 60)pour émettre des signaux ultrasonores depuis différents emplacements de la main de l'opérateur.
